(19) **Europäisches Patentamt European Patent Office Office européen des brevets**

(11) **EP 4 506 057 A1**

(12) **EUROPEAN PATENT APPLICATION**

(43) Date of publication:
**12.02.2025 Bulletin 2025/07**

(21) Application number: **24193241.7**

(22) Date of filing: **07.08.2024**

(51) International Patent Classification (IPC):
**B01F 23/10** (2022.01)     **G01N 33/28** (2006.01)

(52) Cooperative Patent Classification (CPC):
**G01N 33/2829; B01F 23/19**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.08.2023 US 202363519130 P**

(71) Applicant: **Petroleum Analyzer Company, LP Houston, TX 77064 (US)**

(72) Inventors:
• **YOO, Kwang Hee**
  **Houston, TX 77094 (US)**
• **BROSSEAU, Michael**
  **Houston, TX 77008 (US)**
• **HEROLD, Thomas**
  **97944 Boxberg-Uiffingen (DE)**
• **BOYER, Guillaume**
  **64000 Pau (FR)**

(74) Representative: **Zacco Denmark A/S Arne Jacobsens Allé 15 2300 Copenhagen S (DK)**

(54) **SYSTEMS AND METHODS FOR AUTOMATIC CONTROL AND MEASUREMENT OF OXYGEN CONCENTRATION FOR COMBUSTION CONDITIONS**

(57)    The invention provides a gas mixing system (140) comprising: a first conduit (222, 229) to receive nitrogen gas; a second conduit (232, 239) to receive inlet air gas; a gas reservoir (270) coupled to the first conduit (222) and the second conduit (232); a gas mixing manifold (260) coupled to the gas reservoir (260), the first conduit (229), and the second conduit (239); an oxygen sensor (150) coupled to the gas mixing manifold; and a controller (145) configured to: select a target oxygen concentration for a gas mixture for a combustion test; measure, using the oxygen sensor (150), an oxygen concentration in the inlet air gas received via the second conduit (232, 239); control a nitrogen gas flow in the first conduit (222, 229) and an inlet air gas flow in the second conduit (232, 239) to fill the gas reservoir (270) with a gas mixture having the selected target oxygen concentration, based on the measured oxygen concentration in the inlet air gas; and control outlet of the gas mixture from the gas reservoir (270) for use in a combustion test.

FIG. 2

**Description**

BACKGROUND INFORMATION

Hydrocarbon products, such as petroleum and biomass, are used as a source of fuel in combustion engines. Different types of hydrocarbon fuels have different constituents that exhibit different combustion characteristics. Thus, different constituents may affect the performance of a hydrocarbon fuel. A combustion test may be performed on a hydrocarbon fuel sample to determine various properties of the sample. In order for a combustion test to generate accurate results, the conditions under which the combustion test is performed may need to be precisely controlled. Controlling the combustion conditions for a combustion test may pose various difficulties.

BRIEF DESCRIPTION OF THE DRAWINGS

Fig. 1 illustrates a constant volume combustion chamber system according to an implementation described herein;
Fig. 2 illustrates exemplary components of a gas mixing system according to an implementation described herein;
Fig. 3 illustrates an exemplary layout of a gas mixing system according to an implementation described herein;
Fig. 4 illustrates exemplary components of a controller unit and/or other components of a constant volume combustion chamber system according to an implementation described herein;
Fig. 5 illustrates exemplary functional components of a controller unit according to an implementation described herein;
Fig. 6 is a flowchart of a first process for generating and using a gas mixture for a combustion test according to an implementation described herein;
Fig. 7 is a flowchart of a second process for generating and using a gas mixture for a combustion test according to an implementation described herein;
Fig. 8 illustrates an exemplary gas flow for using nitrogen to calibrate an oxygen sensor according to an implementation described herein;
Fig. 9 illustrates an exemplary gas flow for using oxygen to calibrate an oxygen sensor according to an implementation described herein;
Fig. 10 illustrates an exemplary gas flow for measuring the oxygen concentration of an inlet air gas according to an implementation described herein;
Fig. 11 illustrates an exemplary gas flow for generating a gas mixture with a particular oxygen concentration according to an implementation described herein; and
Fig. 12 illustrates an exemplary gas flow for measuring the oxygen concentration of a generated gas mixture according to an implementation described herein.

DETAILED DESCRIPTION OF PREFERRED EMBODIMENTS

The following detailed description refers to the accompanying drawings. The same reference numbers in different drawings identify the same or similar elements.

Research octane number (RON) and Motor Octane Number (MON) are numerical ratings of knock resistance for spark-ignition engine fuels. Knock resistance is a fuel's ability to not self-ignite and burn in an uncontrolled manner while the fuel is being compressed. A RON and MON for a fuel may be determined, for example, by combusting the fuel in a test engine under controlled conditions and comparing the results with the results obtained from a reference mixture of iso-octane and n-heptane (referred to as a primary reference fuel (PRF) blend). A RON may be measured at lower engine speeds (e.g., 600 rpm, etc.), at lower temperatures (e.g., without preheating the fuel mixture, etc.), and/or without using variable ignition timing. A MON may be measured at higher engine speeds (e.g., 900 revolutions per minute (rpm), etc.), at higher temperatures (e.g., with a preheated fuel mixture, etc.), and/or by using variable ignition timing. Another parameter that may be measured in a combustion test may be a cetane number (CN) for a diesel fuel sample, corresponding to a measure of the combustion speed of a diesel fuel sample and the compression needed for ignition.

A combustion test to determine RON, MON, and/or CN may be performed in a standardized single-cylinder Cooperative Fuel Research (CFR) engine. The use of such an engine for performing combustion tests has many drawbacks, such as the requirements for a large volume of fuel per test, a highly trained operator, and large space for the engine itself. Furthermore, the high complexity of the test method that uses a CFR engine exhibits limited repeatability and reproducibility of the measurements, resulting in great expense in formulating standardized fuels.

Instead of using a single-cylinder CFR engine, a combustion test may be performed using a constant volume combustion chamber. A constant volume combustion chamber may use pressure and temperature to create an autoignition event to measure RON, MON, CN, and/or other fuel sample combustion parameters. For example, the constant volume combustion chamber may be used to obtain pressure versus time data and the pressure versus time data may be used to estimate the RON, MON, and/or CN for a fuel sample.

A combustion test may be performed using ambient air to provide the oxygen needed for combustion. However, the amount of oxygen molecules in the air may vary depending on the altitude and barometric pressure of the ambient air. Furthermore, ambient air may include other gases, impurities, and/or pollutants, such as water vapor, volatile hydrocarbons, and/or dust particles, which may affect the results of a combustion test. In particular, the variation of oxygen concentration may have a large impact on the combustion measurements, such as RON, MON, and/or CN. One way to improve the accuracy of a combustion test may be to use synthetic air, which is a mixture of pure nitrogen and pure oxygen in a fixed ratio. However, synthetic air may be expensive to produce and may not provide a high level of accuracy, because the actual oxygen concentration may vary between different canisters of synthetic air.

Implementations described herein relate to systems and methods for automatic control and measurement of oxygen concentration for combustion test conditions. A gas mixing system may enable the generation, storage, and use of a gas mixture with a selected oxygen concentration. An oxygen sensor may measure the oxygen concentration of an inlet air gas and may blend the inlet air gas with an inert gas, such as nitrogen, to generate a gas mixture with a selected oxygen concentration. An inlet air gas may include zero air, stored in a gas storage container, which has had water vapor, carbon dioxide, methane, and/or other impurities removed from air. Inlet air gas may be generated, for example, using a zero-air generator.

The generated gas mixture may be stored in a gas reservoir for use in a combustion test and may be delivered into a constant volume combustion chamber during a combustion test. The gas mixing system may further measure the oxygen concentration of the generated gas mixture to confirm that the gas mixture has an oxygen concentration that is within a specified tolerance of the selected oxygen concentration. Additionally, the gas mixing system may automatically calibrate the oxygen sensor, using nitrogen gas and oxygen gas, to ensure there is no drift or variation in the gas mixing process. Thus, the gas mixing system enables the use of inlet air gas, and/or ambient air, to consistently generate a gas mixture with a selected oxygen concentration, resulting in accurate and consistent combustion tests to determine parameters such as RON, MON, CN, and/or other types of fuel sample combustion parameters.

The gas mixing system may include a first gas conduit to receive nitrogen gas from a nitrogen gas source; a second gas conduit to receive inlet air gas from an inlet air gas source; a third gas conduit to receive oxygen from an oxygen gas source; a gas reservoir coupled to the first gas conduit and the second gas conduit; a gas mixing manifold coupled to the gas reservoir, the first gas conduit, and the second gas conduit; an oxygen sensor coupled to the gas mixing manifold and the third gas conduit; and a controller.

The controller may be configured to select a target oxygen concentration for a gas mixture for a combustion test; measure, using the oxygen sensor, an oxygen concentration in the inlet air gas received via the second gas conduit; control a nitrogen gas flow in the first gas conduit and an inlet air gas flow in the second gas conduit to fill the gas reservoir with a gas mixture having the selected target oxygen concentration, based on the measured oxygen concentration in the inlet air gas; and control the outlet of the gas mixture from the filled gas reservoir for use in a combustion test.

The controller may be further configured to measure, using the oxygen sensor, an oxygen concentration of the gas mixture in the filled gas reservoir to confirm that the gas mixture in the filled gas reservoir has the selected target oxygen concentration.

The gas mixing system may further include a valve to select between the oxygen gas from the third gas conduit or a gas from the gas mixing manifold; and the controller may be further configured to calibrate the oxygen sensor using nitrogen gas from the first gas conduit via the gas mixing manifold to calibrate the oxygen sensor with a zero percentage oxygen concentration gas and to calibrate the oxygen sensor using the oxygen gas from the third gas conduit to calibrate the oxygen sensor with an oxygen concentration gas source having a guaranteed oxygen gas concentration (e.g., 99.994 % oxygen, etc.).

In some implementations, the gas mixing system may include a connection from the third gas conduit to the gas reservoir to enable generation of synthetic air and the controller may be further configured to control the nitrogen gas flow in the first gas conduit and an oxygen gas flow in the third gas conduit to fill the gas reservoir with a synthetic air gas mixture having the selected target oxygen concentration.

The gas mixing system may further include a first mass flow controller to control the flow of nitrogen gas in the first gas conduit; a second mass flow controller to control the flow of inlet air gas in the second gas conduit; and back pressure regulator connected from the first mass flow controller and the second mass controller to the gas reservoir.

The gas mixing system may further include a purge valve connected to the oxygen sensor and configured to purge gas from a conduit connected to the oxygen sensor, to speed up the oxygen measurement process. The gas mixing system may additionally include a pressure regulator connected to the gas reservoir configured to control an outlet pressure for the gas reservoir.

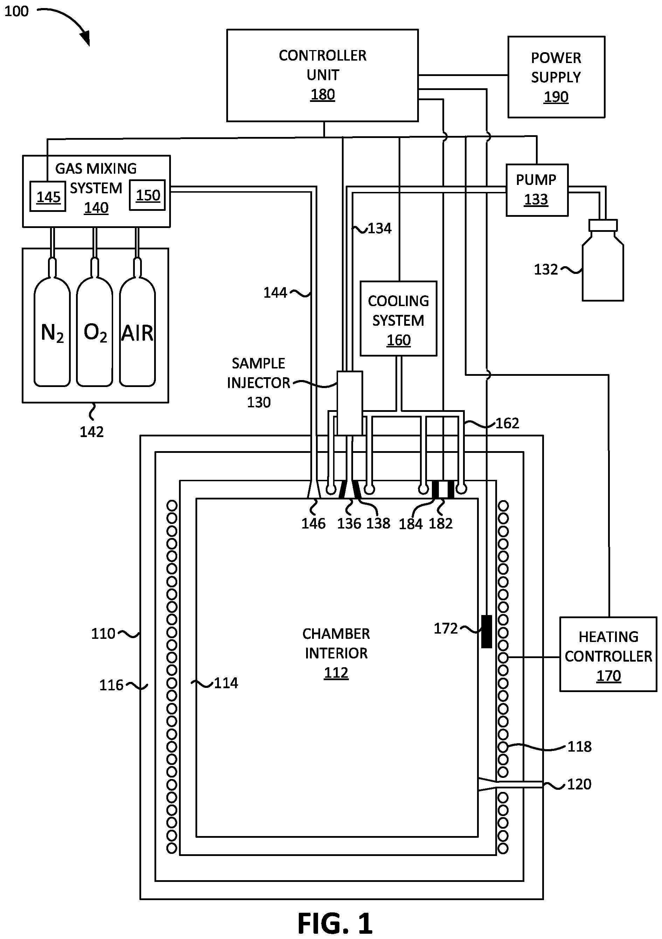

Fig. 1 illustrates a constant volume combustion chamber system 100 according to an implementation described herein. As shown in Fig. 1, constant volume combustion chamber system 100 may include a constant volume combustion chamber 110, a sample injector 130, a sample container 132, a pump 133, a sample conduit 134, a gas mixing system 140, gas cylinders 142, a gas conduit 144, a cooling system 160, cooling system conduits 162, a heating controller 170, a controller unit 180, and a power supply 190.

Constant volume combustion chamber 110 may include a chamber interior 112, inner walls 114, outer walls 116, a heating element 118, an exhaust port 120, sample injector nozzle 136, injector cooling jacket 138, gas conduit nozzle 146, temperature sensor 172, pressure sensor 182, and pressure sensor cooling jacket 184.

Inner walls 114 may enclose chamber interior 112 and outer walls 116 may protect and insulate inner walls 114 and chamber interior 112 from the outside environment. Inner walls 114 and outer walls 116 may enclose heating element 118. Heating element 118 may include a resistive heating element and/or another type of heating element to enable heating controller 170 to raise the temperature of chamber interior 112 to a particular temperature. Exhaust port 120 may enable gases to be removed from chamber interior 112 after a combustion test is completed.

Sample injector nozzle 136 may connect sample conduit 134 to chamber interior 112 and inject a sample into chamber interior 112 for a combustion test. Injector cooling jacket 138 may thermally insulate sample injector nozzle 136 from inner walls 114 to ensure that the sample being injected into chamber interior 112 does not prematurely rise in temperature and to protect sample injector 130 and sample injector nozzle 136 from high temperatures. Gas conduit nozzle 146 may connect gas conduit 144 to chamber interior 112 and inject a gas mixture into chamber interior 112 for a combustion test.

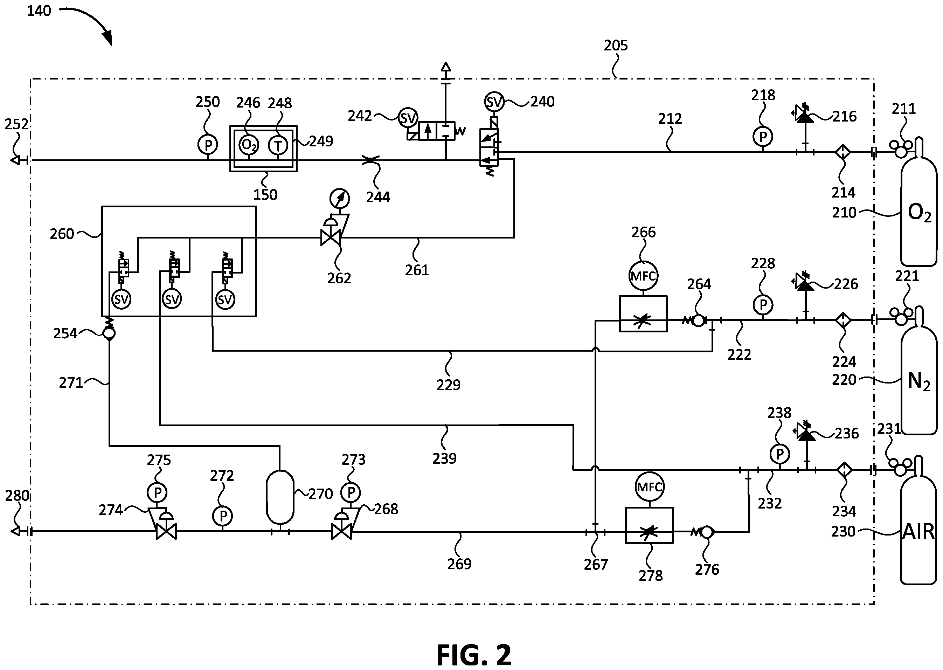

Gas mixing system 140 may mix gases from gas cylinders 142 to generate a particular gas mixture, such as a gas mixture with a specified percentage of oxygen, based on instructions received from controller unit 180. Gas mixing system 140 may store the generated gas mixture in a gas reservoir and deliver the stored gas mixture into chamber interior 112 via gas conduit 144 during a combustion test. Gas cylinders 142 may include an oxygen ($O_2$) gas cylinder, a nitrogen ($N_2$) gas cylinder, and a purified air gas cylinder. Alternatively, ambient air may be used as an air source. In some implementations, gas mixing system 140 may include controller 145. Controller 145 may control the operation of gas mixing system 140. Controller 145 may be in communication with controller unit 180 using a wired and/or wireless connection (e.g., Wi-Fi® connection, Bluetooth® connection, etc.) and may receive instructions from controller unit 180 for controlling the operation of gas mixing system 140, as described in detail below. Gas mixing system 140 may include oxygen sensor 150. Oxygen sensor 150 may measure the oxygen content of a gas in gas mixing system 140, such as an inlet air gas, ambient air, a generated gas mixture, etc. Exemplary components of gas mixing system 140 are described below with reference to Fig. 2.

Temperature sensor 172 may include a thermocouple, a resistance temperature sensor, a thermistor temperature sensor, a semiconductor temperature sensor, and/or another type of temperature sensor. Temperature sensor 172 may measure the temperature of chamber interior 112 and provide the measured temperature to controller unit 180 and/or heating controller 170.

Pressure sensor 182 may measure the pressure in chamber interior 112 during a combustion test. Pressure sensor 182 may include a differential pressure sensor, such as, for example, a diaphragm with a piezoresistive, piezoelectric, and/or capacitive strain gauge. In other implementations, pressure sensor 182 may include another type of pressure sensor, such as an absolute pressure sensor. Pressure sensor cooling jacket 184 may thermally insulate pressure sensor 182 from inner walls 114 to ensure that measurements made by pressure sensor 182 are not affected by the elevated temperatures of inner walls 114.

Sample injector 130 may include a pressure injection system for injecting a fuel sample from sample container 132 into chamber interior 112 via sample conduit 134 and sample injector nozzle 136. Pump 133 may pressurize the sample from sample container 132 and pump the sample to sample injector 130. Cooling system 160 may pump water and/or a cooling fluid through cooling system conduits 162. Cooling system conduits 162 may guide the water and/or cooling fluid to cool sample injector nozzle 136, and/or pressure sensor 182 during a combustion test. In some implementations, pressure sensor 182 may include integrated cooling conduits that may be coupled to cooling system conduits 162 to cool pressure sensor 182 during a combustion test.

Heating controller 170 may be coupled to heating element 118 to provide power to heating element 118 to raise the temperature of chamber interior 112 to a particular temperature during a combustion test. Heating controller 170 may receive information from temperature sensor 172 in order to regulate heating element 118 to maintain a particular temperature in chamber interior 112.

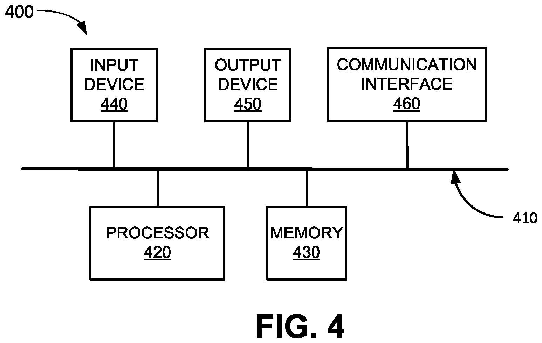

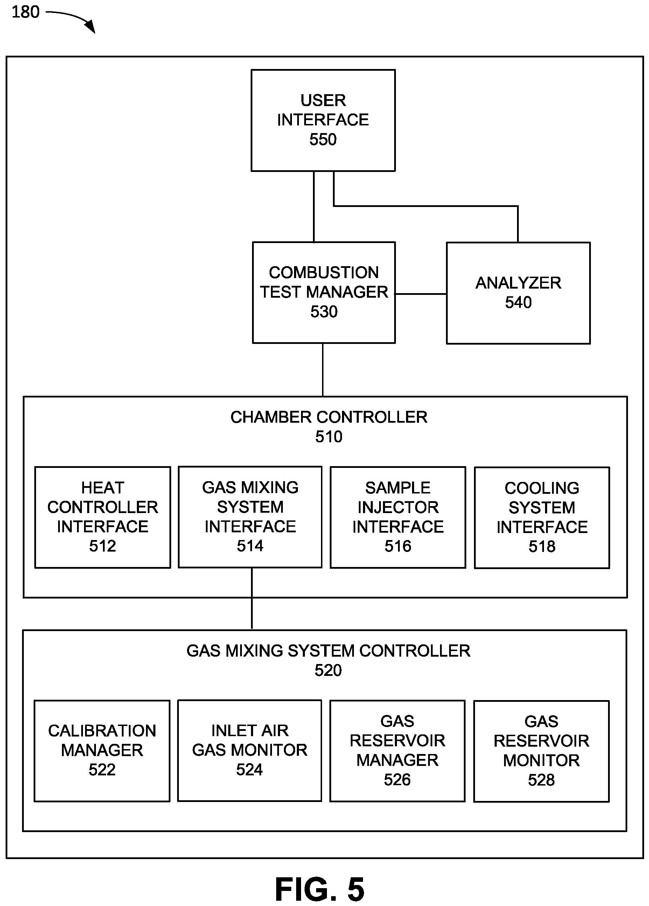

Controller unit 180 may include a processor, microcontroller, and/or computer device that controls the operation of constant volume combustion chamber system 100, including gas mixing system 140. Exemplary components of controller unit 180 are described below with reference to Figs. 4 and 5. Power supply 190 may supply power to controller unit 180 and to sample injector 130, gas mixing system 140, oxygen sensor 150, cooling system 160, heating controller 170, and/or pressure sensor 182 (connections not shown in Fig. 1). In other implementations, gas mixing system 140 may include a separate controller (not shown in Fig. 1) that receives instructions from controller unit 180.

Although Fig. 1 shows exemplary components of constant volume combustion chamber system 100, in other implementations, constant volume combustion chamber system 100 may include fewer components, different components, differently arranged components, or additional components than depicted in Fig. 1. Additionally, or alternatively, one or more components of constant volume combustion chamber system 100 may perform functions described as being performed by one or more other components of constant volume combustion chamber system 100.

Fig. 2 illustrates exemplary components of gas mixing system 140. As shown in Fig. 2, gas mixing system 140 may include an enclosure 205, an oxygen gas source 210, an oxygen gas regulator 211, an oxygen gas conduit 212, a filter 214, a safety relief valve 216, a pressure sensor 218, a nitrogen gas source 220, a nitrogen gas regulator 221, a nitrogen gas conduit 222, a filter 224, a safety relief valve 226, a pressure sensor 228, a second nitrogen gas conduit 229, an inlet air gas source 230, an inlet air gas regulator 231, an inlet air gas conduit 232, a filter 234, a pressure relief valve 236, a pressure sensor 238, a second inlet air gas conduit 239, a gas selection valve 240, a purge valve 242, a flow control valve 244, oxygen sensor 150, pressure sensor 250, a measured gas outlet 252, a check valve 254, a gas mixing manifold 260, a gas mixing manifold conduit 261, a regulator 262, a check valve 264, a mass flow controller (MFC) 266, a T interconnection 267, a back pressure regulator 268, a gas reservoir inlet conduit 269, a gas reservoir 270, a gas reservoir outlet conduit 271, a pressure sensor 272, a pressure sensor 273, a regulator 274, a pressure gauge 275, a check valve 276, a mass flow controller 278, and a gas reservoir outlet 280. Controller 145 may be connected to some or all sensors and actuators of gas mixing system 140 via wired and/or wireless connections. For illustrative purposes, controller 145 and the connections between controller 145 and the sensors and actuators of gas mixing system 140 are not shown in Fig. 2.

Enclosure 205 may enclose and house the components of gas mixing system 140 and protect the components of gas mixing system 140 from the environment. In some implementations, enclosure 205 may include a metal, plastic, wood, and/or composite container that fully or partially encloses the components of gas mixing system 205 and protects the components of gas mixing system 140 from dust and/or other contaminants. Additionally, in some implementations, enclosure 205 may be water resistant, waterproof, and/or thermally insulating. Oxygen gas conduit 212, nitrogen gas conduit 222, inlet air gas conduit 232, purge valve 242, measured gas outlet 252, and/or gas reservoir outlet 280 may be mounted to the walls of enclosure 205 with bulkheads. In other implementations, enclosure 205 may include a partially open or fully open structural chassis, such as a rectangular chassis with or without diagonal support beams. The chassis may include metal and/or plastic structural tubing with rectangular, H-profile, C-profile, and/or L-profile cross-sections; solid bar beams; and/or other types of beams.

Oxygen gas source 210 may include a gas canister, cylinder, and/or tank of oxygen with a guaranteed oxygen concentration (e.g., 99.994% oxygen, etc.). Oxygen gas regulator 211 may control the pressure of oxygen gas from oxygen gas source 210. For example, oxygen gas regulator 211 may be set to 2.8 bar. Oxygen gas conduit 212 may include a gas conduit from oxygen gas source 210 to gas selection valve 240. Oxygen gas conduit 212 may include, for example, 1/8-inch diameter polytetrafluoroethylene (PFE) tubing. In some implementations, oxygen gas conduit 212 may include a mass flow controller and a connection (not shown in Fig. 2) to gas reservoir 270 to enable generation of a synthetic air gas mixture and/or to generate a gas mixture with a higher oxygen concentration than the oxygen concentration of inlet air gas source 230. Filter 214 may filter out particulates from oxygen gas source 210. For example, filter 214 may include a two micron filter. Safety relief valve 216 may be configured to release pressure in oxygen gas conduit 212 if the pressure rises above a threshold value. For example, safety relief valve 216 may be set to 3.5 bar and may open when the pressure reaches 3.5 bar. Pressure sensor 218 may monitor pressure inside oxygen gas conduit 212.

Nitrogen gas source 220 may include a gas canister, cylinder, and/or tank of nitrogen with a guaranteed nitrogen concentration (e.g., 99.999% nitrogen, etc.). In other implementations, a different inert gas may be used (e.g., argon, etc.). Nitrogen gas regulator 221 may control the pressure of nitrogen gas from nitrogen gas source 220. For example, nitrogen gas regulator 221 may be set to 40 bar. Nitrogen gas conduit 222 may include a gas conduit from nitrogen gas source 220 to T interconnection 267. Nitrogen gas conduit 222 may include, for example, 1/4-inch diameter polyamide (PA) tubing. A second nitrogen gas conduit 229 may connect nitrogen gas source 220 to gas mixing manifold 260. Second nitrogen gas conduit 229 may include, for example, 1/8-inch diameter PA tubing. Filter 224 may filter out particulates from nitrogen gas source 220. For example, filter 224 may include a two micron filter that filters particles greater than two microns in diameter. Safety relief valve 226 may be configured to release pressure in nitrogen gas conduit 222 if the pressure rises above a threshold value. For example, safety relief valve 226 may be set to 44 bar and may open when the pressure reaches 44 bar. Pressure sensor 228 may monitor pressure inside nitrogen gas conduit 222.

Inlet air gas source 230 may include a gas canister, cylinder, and/or tank of zero air. In other implementations, compressed air may be used as a source of inlet air gas. Furthermore, in some implementations, gas mixing system 140 may include a gas selection valve (not shown in Fig. 2) to enable selection between a zero air gas source and compressed air. Inlet air gas regulator 231 may control the pressure of inlet air gas from inlet air gas source 230. For example, inlet air gas regulator 231 may be set to 40 bar. Inlet air gas conduit 232 may include a gas conduit from inlet air gas source 230 to T interconnection 267. Inlet air gas conduit 232 may include, for example, 1/4-inch diameter PA tubing. A second inlet air gas conduit 239 may connect inlet air gas source 230 to gas mixing manifold 260. Second inlet air gas conduit 239 may include, for example, 1/8-inch diameter PA tubing. Filter 234 may filter out particulates from inlet air gas source 230. For example, filter 234 may include a two micron filter. Safety relief valve 236 may be configured to release pressure in inlet air gas conduit 232 if the pressure rises above a threshold value. For example, safety relief valve 236 may be set to 44 bar. Pressure sensor 238 may monitor pressure inside inlet air gas conduit 232.

Gas selection valve 240 may include a solenoid-activated self-return valve configured to select gas flow between oxygen gas in oxygen gas conduit 212 or gas from gas mixing manifold conduit 261. Purge valve 242 may include a solenoid-activated self-return valve that functions to purge gas from the conduit to oxygen sensor 150. Purge valve 242 may speed up oxygen concentration measurements in gas mixing system 140. Flow control valve 244 may reduce the diameter of the gas conduit input into oxygen sensor 150 to 75 microns. Oxygen sensor 150 may include an oxygen sensor module 246, a temperature sensor 248, and a heater 249. Oxygen sensor module 246 may be calibrated at a particular temperature (e.g., at 55 degrees °C, etc.). Temperature sensor 248 may measure the temperature of oxygen sensor module 246 and controller 145 may use heater 249 and the temperature measurements made by temperature sensor 248 to maintain oxygen sensor module 246 at the particular temperature.

Pressure sensor 250 may measure the pressure in the measured gas outlet conduit. Measured gas outlet 252 may outlet gas being measured by oxygen sensor 150 to enable flow of gas through oxygen sensor 150. The measured pressure in the gas outlet conduit may be used to perform pressure compensation on the oxygen concentration measured by oxygen sensor 150. Oxygen sensor 150 may be calibrated at a calibration pressure and an oxygen concentration measured by oxygen sensor 150 may be adjusted based on a ratio of the calibrated pressure to the pressure measured by pressure sensor 250. The adjusted oxygen concentration may be computed using the following equation: $O_A = O_M*(P_C/P_M)$, where $O_A$ corresponds to the adjusted oxygen concentration, $O_M$ corresponds to the measured oxygen concentration, Pc corresponds to the calibration pressure, and $P_M$ corresponds to the pressure measured by pressure sensor 250.

Check valve 254 may include a spring return check valve to prevent backflow of gas in gas reservoir outlet conduit 271. Gas mixing manifold 260 may include a first inlet from second nitrogen gas conduit 229, a second inlet from second inlet air gas conduit 239, a third inlet from gas reservoir outlet conduit 271, and an outlet to gas mixing manifold conduit 261. Each inlet of gas mixing manifold 260 may include a solenoid-activated self-return valve. Operation of gas mixing manifold 260 by controller 145 (e.g., by itself or together with controller unit 180, etc.) enables selection of a gas from the first inlet, second inlet, or third inlet to outlet gas mixing manifold conduit 261. Thus, gas mixing manifold 260 enables selection of outlet gas as nitrogen gas from nitrogen gas source 220, inlet air gas from inlet air gas source 230, or stored gas mixture from gas reservoir 270 as the inlet gas into oxygen sensor 150 in order to measure the oxygen concentration of the outlet gas from gas mixing manifold 260. Regulator 262 may include a needle valve and a pressure gauge configured to maintain a particular pressure in gas mixing manifold conduit 261. For example, regulator 262 may be set to 2.9 bar.

Check valve 264 may include a spring return check valve to prevent backflow of gas in nitrogen gas conduit 222. Mass flow controller 266 may control the flow of gas in nitrogen gas conduit 222. Check valve 276 may include a spring return check valve to prevent backflow of gas in inlet air gas conduit 232. Mass flow controller 278 may control the flow of gas in inlet air gas conduit 232. T interconnection 267 may interconnect nitrogen gas conduit 222 and inlet air gas conduit 232 with gas reservoir inlet conduit 269. Back pressure regulator 268 may maintain a particular pressure in gas reservoir inlet conduit 269 based on pressure measured by pressure sensor 273. For example, back pressure regulator 268 may be set to a particular pressure (e.g., 28 bar, etc.) to maintain an optimal pressure difference between the inlet and outlet of MFC 266 and MFC 278 in order to maintain proper flow control. Gas reservoir inlet conduit 269 may connect T interconnection 267 with the inlet of gas reservoir 270.

Gas reservoir 270 may include a reservoir to store a gas mixture generated by mixing nitrogen gas from nitrogen gas source 220 and inlet air gas from inlet air gas source 230. In some implementations, gas reservoir inlet conduit 269 may further include a connection from oxygen gas conduit 212 to enable generation of a synthetic air gas mixture. Gas reservoir 270 may include, for example a one-liter gas reservoir. Gas reservoir outlet conduit 271 may interconnect gas reservoir 270 with an inlet of gas mixing manifold 260 to enable the measurement of the oxygen concentration of the gas mixture stored in gas reservoir 270. Pressure sensor 272 may measure the pressure in gas reservoir 270 in order to monitor the quantity of mixed gas in gas reservoir 270. Regulator 274 may maintain a particular pressure in gas reservoir outlet 280 based on pressure measured by pressure gauge 275. For example, regulator 274 may be set to 25 bar to ensure that the gas delivered to chamber interior 112 during a combustion test is delivered at a pressure of 25 bar. Gas reservoir outlet 280 may provide gas from gas reservoir 270 to chamber interior 112 during a combustion test. While gas reservoir outlet 280 is shown as being connected to gas reservoir inlet conduit 269 via a T interconnect, in other implementations, gas reservoir outlet 280 may be connected to gas reservoir outlet conduit 271 with a T interconnect.

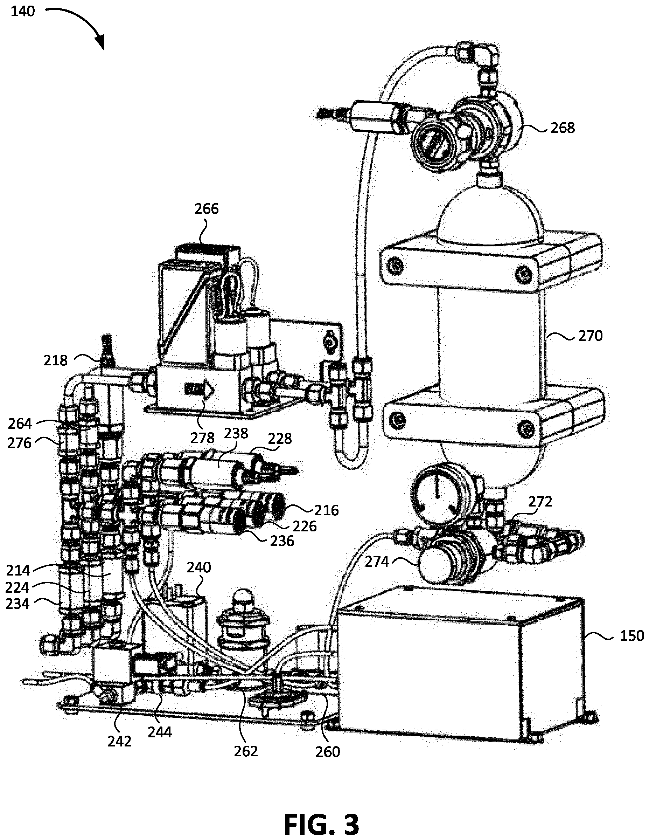

Fig. 3 illustrates an exemplary layout of gas mixing system 140. As mentioned above, in some implementations, the components of gas mixing system 140 may be mounted on an open chassis. In other implementations, the components of gas mixing system 140 may be mounted inside a closed enclosure and gas inlets and outlets, such as oxygen gas conduit 212, nitrogen gas conduit 222, inlet air gas conduit 232, purge valve 242, measured gas outlet 252, and/or gas reservoir outlet 280 may be mounted to the walls of the enclosure with bulkheads. For illustrative purposes, an open chassis and/or enclosure is not shown in Fig. 3.

Although Figs. 2 and 3 shows exemplary components of gas mixing system 140, in other implementations, gas mixing system 140 may include fewer components, different components, differently arranged components, or additional components than depicted in Figs. 2 or 3. Additionally, or alternatively, one or more components of gas mixing system 140 may perform functions described as being performed by one or more other components of gas mixing system 140.

Fig. 4 is a diagram illustrating example components of device 400 according to an implementation described herein. Sample injector 130, gas mixing system 140, cooling system 160, heating controller 170, and/or controller unit 180 may each include one or more devices 400. As shown in Fig. 4, device 400 may include a bus 410, a processor 420, a memory 430, an input device 440, an output device 450, and a communication interface 460.

Bus 410 may include a path that permits communication among the components of device 400. Processor 420 may include any type of single-core processor, multi-core processor, microprocessor, latch-based processor, and/or processing logic (or families of processors, microprocessors, and/or processing logics) that interprets and executes instructions. In other embodiments, processor 420 may include an application-specific integrated circuit (ASIC), a field-programmable gate array (FPGA), and/or another type of integrated circuit or processing logic.

Memory 430 may include any type of dynamic storage device that may store information and/or instructions, for execution by processor 420, and/or any type of non-volatile storage device that may store information for use by processor 420. For example, memory 430 may include a random access memory (RAM) or another type of dynamic storage device, a read-only memory (ROM) device or another type of static storage device, a content addressable memory (CAM), a magnetic and/or optical recording memory device and its corresponding drive (e.g., a hard disk drive, optical drive, etc.), and/or a removable form of memory, such as a flash memory.

Input device 440 may allow an operator to input information into device 400. Input device 440 may include, for example, a keyboard, a mouse, a pen, a microphone, a remote control, an audio capture device, an image and/or video capture device, a touch-screen display, and/or another type of input device. In some embodiments, device 400 may be managed remotely and may not include input device 440. In other words, device 400 may be "headless" and may not include a keyboard, for example.

Output device 450 may output information to an operator of device 400. Output device 450 may include a display, a printer, a speaker, and/or another type of output device. For example, device 400 may include a display, which may include a liquid-crystal display (LCD), light emitting diode (LED) display, etc., for displaying content to the operator. In some embodiments, device 400 may be managed remotely and may not include output device 450. In other words, device 400 may be "headless" and may not include a display, for example.

Communication interface 460 may include a transceiver that enables device 400 to communicate with other devices and/or systems via wireless communications (e.g., radio frequency, infrared, and/or visual optics, etc.), wired communications (e.g., conductive wire, twisted pair cable, coaxial cable, transmission line, fiber optic cable, and/or waveguide, etc.), or a combination of wireless and wired communications. Communication interface 460 may include a transmitter that converts baseband signals to radio frequency (RF) signals and/or a receiver that converts RF signals to baseband signals. Communication interface 460 may be coupled to an antenna for transmitting and receiving RF signals.

Communication interface 460 may include a logical component that includes input and/or output ports, input and/or output systems, and/or other input and output components that facilitate the transmission of data to other devices. For example, communication interface 460 may include a network interface card (e.g., Ethernet card) for wired communications and/or a wireless network interface (e.g., a WiFi) card for wireless communications. Communication interface 460 may also include a universal serial bus (USB) port for communications over a cable, a Bluetooth™ wireless interface, a radio-frequency identification (RFID) interface, a near-field communications (NFC) wireless interface, and/or any other type of interface that converts data from one form to another form.

As will be described in detail below, device 400 may perform certain operations relating to generating, storing, and/or using a gas mixture with a specified oxygen concentration for a combustion test. Device 400 may perform these operations in response to processor 420 executing software instructions contained in a computer-readable medium, such as memory 430. A computer-readable medium may be defined as a non-transitory memory device. A memory device may be implemented within a single physical memory device or spread across multiple physical memory devices. The software instructions may be read into memory 430 from another computer-readable medium or from another device. The software instructions contained in memory 430 may cause processor 420 to perform processes described herein. Alternatively, hardwired circuitry may be used in place of, or in combination with, software instructions to implement processes described herein. Thus, implementations described herein are not limited to any specific combination of hardware circuitry and software.

Although Fig. 4 shows exemplary components of device 400, in other implementations, device 400 may include fewer components, different components, additional components, or differently arranged components than depicted in Fig. 4. Additionally, or alternatively, one or more components of device 400 may perform one or more tasks described as being performed by one or more other components of device 400.

Fig. 5 illustrates exemplary functional components of controller unit 180 and/or controller 145. In some implementations, the functional components of Fig. 5 may be implemented in controller unit 180. In other implementations, one or more of the functional components of Fig. 5 may be implemented in controller 145 and the other functional components of Fig. 5 may be implemented in controller unit 180. The functional components of controller unit 180 and/or controller 145 may be implemented, for example, via processor 420 executing instructions from memory 430. As shown in Fig. 5, controller unit 180 and/or controller 145 may include a chamber controller 510, a gas mixing system controller 520, a combustion test manager 530, an analyzer 540, and a user interface 550.

Chamber controller 510 may control the operation of constant volume combustion chamber system 100 based on information received from combustion test manager 530. Chamber controller 510 may include a heating controller interface 512, a gas mixing system interface 514, a sample injector interface 516, and a cooling system interface 518. Heating controller interface 512 may be configured to interface with heating controller 170. For example, heating controller interface 512 may instruct heating controller 170 to maintain a particular temperature during a combustion test. Gas mixing system interface 514 may be configured to interface with gas mixing system controller 520. For example, gas mixing system interface 514 may instruct gas mixing system controller 520 to generate a particular gas mixture for a combustion test (e.g., a gas mixture with a particular oxygen percentage) and/or generate a particular pressure inside chamber interior 112 for the combustion test. Sample injector interface 516 may be configured to interface with sample injector 130. For example, sample injector interface 516 may instruct sample injector 130 to inject a sample at a particular duration during a combustion test. Cooling system interface 518 may be configured to interface with cooling system 160. For example, cooling system interface 518 may instruct cooling system 160 to start circulating water and/or cooling fluid before, during, and/or after a combustion test.

Gas mixing system controller 520 may control the operation of gas mixing system 140. In some implementations, gas mixing system controller 520 may be included in controller unit 180. In other implementations, gas mixing system controller 520 may be included in controller 145. Gas mixing system controller 520 may include a calibration manager 522, an inlet air gas monitor 524, a gas reservoir manager 526, and a gas reservoir monitor 528.

Calibration manager 522 may calibrate oxygen sensor 150 at particular intervals. For example, calibration manager 522 may calibrate oxygen sensor 150 before each combustion test. Calibration manager 522 may use nitrogen gas from nitrogen gas source 220 to calibrate oxygen sensor 150 for a zero-percentage oxygen concentration gas or a guaranteed or predetermined maximum oxygen concentration for nitrogen gas source 220. Calibration manager 522 may use oxygen gas from oxygen gas source 210 to calibrate oxygen sensor 150 with an oxygen concentration gas source having a guaranteed minimum oxygen gas concentration (e.g., 99.994 % oxygen, etc.).

Inlet air gas monitor 524 may monitor the oxygen concentration in inlet air gas from inlet air gas source 230 and/or in ambient air. For example, inlet air gas monitor 524 may use gas mixing manifold 260 to select the gas from second inlet air gas conduit 239 as the outlet gas in gas mixing manifold conduit 261 and may control gas selection valve 240 to select the gas from gas mixing manifold conduit 261 as the inlet gas for oxygen sensor 150.

Gas reservoir manager 526 may manage the gas mixture in gas reservoir 270. For example, gas reservoir manager 526 may control mass flow controllers 266 and 278 and backpressure regulator 268 to fill gas reservoir 270 with a gas mixture having a selected oxygen concentration (e.g., 20% oxygen, etc.) and a selected pressure (e.g., 35 bar, etc.). Furthermore, gas reservoir manager 526 may control the outlet of gas from gas reservoir 270 to chamber interior 112 via gas reservoir outlet 280 at a particular pressure (e.g., 25 bar, etc.).

Gas reservoir monitor 528 may monitor the oxygen concentration in the gas mixture in gas reservoir 270. For example, gas reservoir monitor 528 may use gas mixing manifold 260 to select the gas from gas reservoir outlet conduit 271 as the outlet gas in gas mixing manifold conduit 261 and may control gas selection valve 240 to select the gas from gas mixing manifold conduit 261 as the inlet gas for oxygen sensor 150.

Combustion test manager 530 may manage combustion tests performed by constant volume combustion chamber system 100. For example, combustion test manager 530 may obtain a combustion test setting from a set of stored combustion test settings, perform a combustion test according to the obtained combustion test setting, receive pressure versus time data for the combustion test, and store the received pressure versus time data in a database.

Analyzer 540 may use the determined pressure parameter values for samples for which combustion parameters are known to generate a combustion parameter function for a set of combustion test conditions. Analyzer 540 may correlate a set of pressure parameter values for a sample and the known or previously determined parameter values for the sample to generate a parameter function using regression analysis and/or a machine learning model.

User interface 550 may be configured to interact with input device 440 and/or output device 450. User interface 550 may include an interface that enables a user to control constant volume combustion chamber system 100 and/or receive information generate by controller unit 180, such as a generated pressure versus time plot, a plot of a particular pressure parameter derived from pressure versus time data, a generated parameter function, a computed parameter for a sample, messages regarding a combustion test that has been completed or is in progress, and/or other types of information that may be inputted by a user or that may be outputted for the user. Additionally, user interface 550 may be used to control and/or configure gas mixing system 140. For example, user interface 550 may be used to instruct gas mixing system controller 520 to select an oxygen concentration for a gas mixture, to calibrate oxygen sensor 150, to measure the oxygen concentration of a inlet air gas source, to measure the oxygen concentration of a gas mixture in gas reservoir 270, to set a pressure threshold for a particular regulator in gas mixing system 140, and/or to perform another function or configuration associated with gas mixing system 140.

Although Fig. 5 shows exemplary components of controller unit 180 and/or controller 145, in other implementations, controller unit 180 and/or controller 145 may include fewer components, different components, additional components, or differently arranged components than depicted in Fig. 5. Additionally, or alternatively, one or more components of controller unit 180 and/or controller 145 may perform one or more tasks described as being performed by one or more other components of controller unit 180 and/or controller 145.

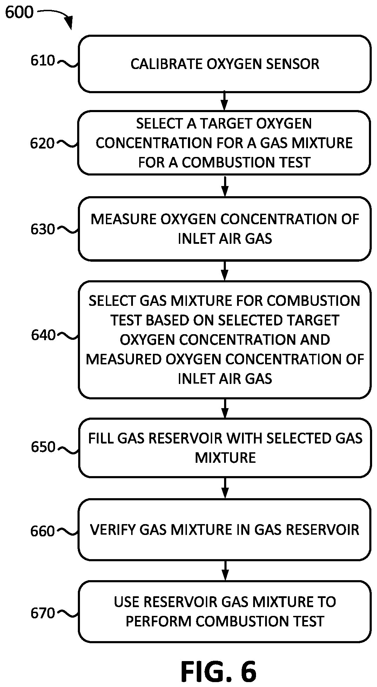

Fig. 6 is a flowchart of a first process 600 for generating and using a gas mixture for a combustion test according to an implementation described herein. In some implementations, the process of Fig. 6 may be performed by, and/or using, constant volume combustion chamber system 100. In other implementations, some or all of the process of Fig. 6 may be performed by, or using, another device or a group of devices separate from constant volume combustion chamber system 100.

Process 600 may include calibrating an oxygen sensor (block 610). For example, controller unit 180 and/or controller 145 may control gas mixing system 140 to measure the oxygen concentration of nitrogen gas source 220 using oxygen sensor 150 and then adjust the measured oxygen concentration to the known oxygen concentration of nitrogen gas source 220. The known oxygen concentration of nitrogen gas source 220 may be based on a guaranteed or predetermined maximum oxygen concentration for nitrogen gas source 220. Controller unit 180 and/or controller 145 may then control gas mixing system 140 to measure the oxygen concentration of oxygen gas source 210 using oxygen sensor 150 and then adjust the measured oxygen concentration to the known oxygen concentration of oxygen gas source 210. The known oxygen concentration of oxygen gas source 210 may be based on a guaranteed or predetermined minimum oxygen concentration for oxygen gas source 210. Alternatively, the oxygen concentration of oxygen gas source 210 may be measured during calibration, followed by measuring the oxygen concentration of nitrogen gas source 220.

Process 600 may further include selecting a target oxygen concentration for a gas mixture for a combustion test (block 620), measuring the oxygen concentration of an inlet air gas (block 630), selecting a gas mixture for a combustion test based on the selected target oxygen concentration and the measured oxygen concentration of the inlet air gas (block 640), and filling a gas reservoir with the selected gas mixture (block 650).

For example, an operator may select an oxygen concentration for a combustion test (e.g., 20% oxygen, etc.). Controller unit 180 and/or controller 145 may control gas mixing system 140 to measure the oxygen concentration of inlet air gas source 230 (and/or the oxygen concentration of ambient air) using oxygen sensor 150. Controller unit 180 and/or controller 145 may then determine how to generate a gas mixture with the selected oxygen concentration based on the determined oxygen concentration of the inlet air gas (and/or the oxygen concentration of the ambient air). For example, controller unit 180 and/or controller 145 may determine how much nitrogen gas to add to the inlet air gas (and/or ambient air) to reduce the oxygen concentration of the inlet air gas (and/or ambient air) to the selected oxygen concentration. Gas reservoir manager 526 may maintain a database that relates a target oxygen concentration and the oxygen concentration of inlet air gas source 230 to control settings of mass flow controller 266, mass flow controller 278, and/or back pressure regulator 268 to arrive at a gas mixture with the target oxygen concentration. Gas mixing system 140 may fill gas reservoir 270 with the gas mixture. Gas mixing system 140 may then measure the oxygen concentration of the gas mixture in gas reservoir 270 to ensure that the gas mixture has the target oxygen concentration.

In some implementations, in addition to being able to reduce the oxygen concentration of inlet air gas source 230 to a target oxygen concentration using nitrogen gas source 220, gas mixing system 140 may be configured to increase the oxygen concentration of inlet air gas source 230 to a target oxygen concentration using oxygen gas source 210. For example, gas mixing system 140 may include a gas conduit, from oxygen gas conduit 212 to gas reservoir inlet conduit 269, which includes a mass flow controller and a check valve.

Process 600 may further include verifying the gas mixture in the gas reservoir (block 660) and using the reservoir gas mixture to perform a combustion test (block 670). Gas mixing system 140 may measure an oxygen concentration of the gas mixture in gas reservoir 270 to determine whether the gas mixture in gas reservoir 270 has the selected target oxygen concentration. For example, gas mixing system 140 may control gas mixing manifold 260 to outlet the reservoir gas mixture to gas mixing manifold conduit 261 and gas selection valve 240 to select the reservoir gas mixture from gas mixing manifold conduit 261 through oxygen sensor 150 to measure the oxygen concentration and/or total pressure in gas reservoir 270. If the oxygen concentration and/or total pressure in gas reservoir 270 corresponds to the selected oxygen concentration and/or total pressure, gas mixing system 140 may proceed with the combustion test by controlling regulator 274 to deliver the gas mixture from gas reservoir 270 to chamber interior 112 during a combustion test. If the oxygen concentration and/or total pressure in gas reservoir 270 does not correspond to the selected oxygen concentration and/or total pressure, gas mixing system 140 may not perform the combustion test and may advise the operator to check the gas reservoir filling process.

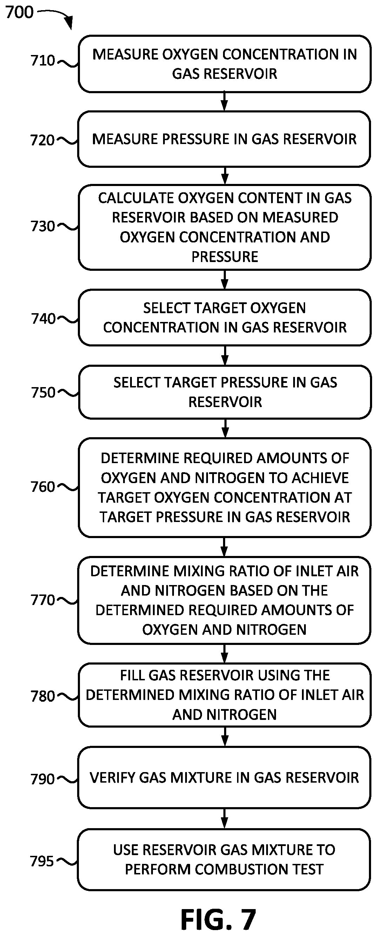

Fig. 7 is a flowchart of a second process 700 for generating and using a gas mixture for a combustion test. In some implementations, the process of Fig. 7 may be performed by, and/or using, constant volume combustion chamber system 100. In other implementations, some or all of the process of Fig. 7 may be performed by, or using, another device or a group of devices separate from constant volume combustion chamber system 100.

Process 700 may include measuring oxygen concentration in a gas reservoir (block 710), measuring the pressure in the gas reservoir (block 720), and calculating the oxygen content in the gas reservoir based on the measured oxygen concentration and pressure (block 730). Controller unit 180 and/or controller 145 may control gas mixing system 140 to measure the oxygen concentration of gas reservoir 270 using oxygen sensor 150 and use pressure sensor 272 to measure the pressure in gas reservoir 270. Controller unit 180 and/or controller 145 may then calculate the oxygen and nitrogen mole numbers of gas reservoir 270 based on the measured pressure and oxygen concentration using, for example, the ideal gas law and the law of partial pressures. For example, the oxygen mole number of gas reservoir 270 may be calculated as $n_O = \dfrac{C_O * P * V}{R * T}$, where no corresponds to the oxygen mole number, Co corresponds to the measured oxygen concentration, P corresponds to the measured pressure, V corresponds to the volume of gas reservoir 270, R corresponds to the ideal gas constant, and T corresponds to the temperature of gas reservoir 270.

Process 700 may further include selecting a target oxygen concentration in the gas reservoir (block 740) and selecting a target pressure in the gas reservoir (block 750). For example, an operator may select an oxygen concentration for a combustion test (e.g., 20% oxygen, etc.) and a pressure in gas reservoir 270 to deliver the gas mixture during the combustion test at a target pressure. The pressure in gas reservoir 270 may be selected so that control regulator 274 may deliver the gas mixture at the target pressure. For example, the pressure in gas reservoir 270 may be selected to be higher by at least a threshold amount than the target pressure.

Process 700 may further include determining the required amounts of oxygen and nitrogen to achieve the target oxygen concentration at the target pressure in the gas reservoir (block 760) and determining a mixing ratio of inlet air and nitrogen based on the determined required amounts of oxygen and nitrogen (block 770). For example, controller unit 180 and/or controller 145 may determine the required amount of oxygen and nitrogen to generate a gas mixture in gas reservoir 270 with the target oxygen concentration and target pressure based on the calculated amount of oxygen and nitrogen already in gas reservoir 270. Controller unit 180 and/or controller 145 may determine the ratio of inlet air and nitrogen based on the required amount of oxygen and nitrogen. Controller unit 180 and/or controller 145 may then set the flow rates of MFC 266 and MFC 278 based on the determined ratio.

Process 700 may further include filling the gas reservoir with the determined mixing ratio of inlet air and nitrogen (block 780). For example, gas reservoir manager 526 may control settings of MFC 266, MFC 278, and/or back pressure regulator 268 to arrive at a gas mixture with the target oxygen concentration and target pressure using the determined mixing ratio. Gas mixing system 140 may fill gas reservoir 270 with the gas mixture. Gas mixing system 140 may then measure the oxygen concentration of the gas mixture in gas reservoir 270 to ensure that the gas mixture has the target oxygen concentration. Furthermore, gas mixing system 140 may measure the pressure within gas reservoir 270 to ensure that gas reservoir 270 has been filled to at least the target pressure.

Process 700 may further include verifying the gas mixture in the gas reservoir (block 790) and using the reservoir gas mixture to perform a combustion test (block 795). Gas mixing system 140 may measure an oxygen concentration of the gas mixture in gas reservoir 270 to determine whether the gas mixture in gas reservoir 270 has the selected target oxygen concentration. For example, gas mixing system 140 may control gas mixing manifold 260 to outlet the reservoir gas mixture to gas mixing manifold conduit 261 and gas selection valve 240 to select the reservoir gas mixture from gas mixing manifold conduit 261 through oxygen sensor 150 to measure the oxygen concentration and/or total pressure in gas reservoir 270. If the oxygen concentration and/or total pressure in gas reservoir 270 corresponds to the selected oxygen concentration and/or total pressure, gas mixing system 140 may proceed with the combustion test by controlling regulator 274 to deliver the gas mixture from gas reservoir 270 to chamber interior 112 during a combustion test. If the oxygen concentration and/or total pressure in gas reservoir 270 does not correspond to the selected oxygen concentration and/or total pressure, gas mixing system 140 may not perform the combustion test and may advise the operator to check the gas reservoir filling process.

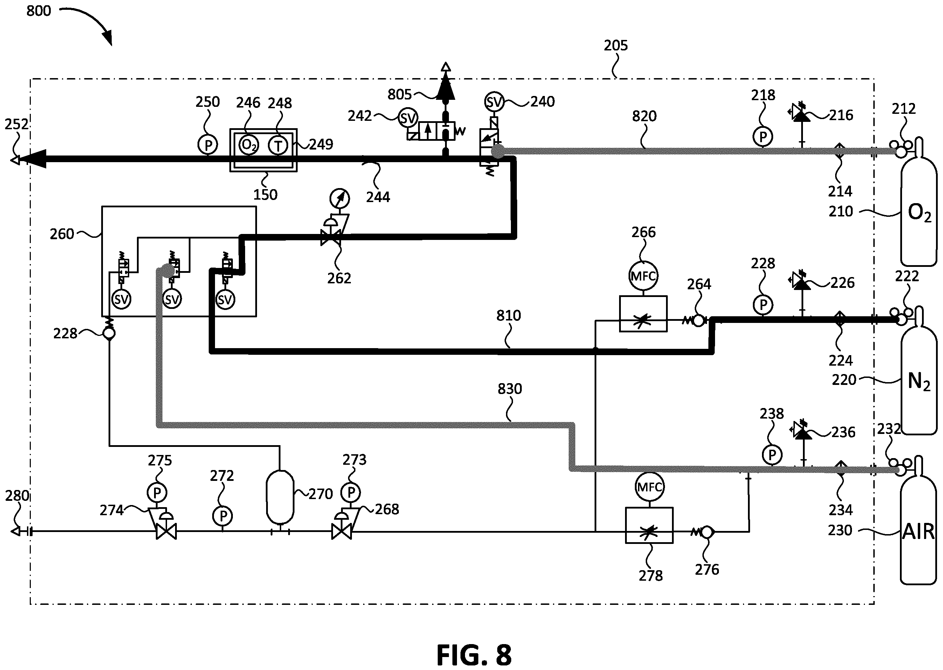

Fig. 8 illustrates an exemplary gas flow 800 for using nitrogen to calibrate an oxygen sensor according to an implementation described herein. As shown in Fig. 8, before any oxygen measurements are made, gas flow 800 includes using purge flow 805 to purge any residual gases from oxygen sensor 150, oxygen gas conduit 212, gas selection valve 240, gas mixing manifold 260, and/or gas mixing manifold conduit 261 via purge valve 242. Gas flow 800 subsequently includes nitrogen gas flow 810 from nitrogen gas source 220 via second nitrogen gas conduit 229 to gas mixing manifold 260. Gas mixing manifold 260 may be controlled to outlet the nitrogen gas to gas mixing manifold conduit 261 and gas selection valve 240 may be controlled to select the nitrogen gas from gas mixing manifold conduit 261 through oxygen sensor 150 to measured gas outlet 252. Oxygen gas flow 820 may terminate at gas selection valve 240. Inlet air gas flow 830 may terminate at gas mixing manifold 260.

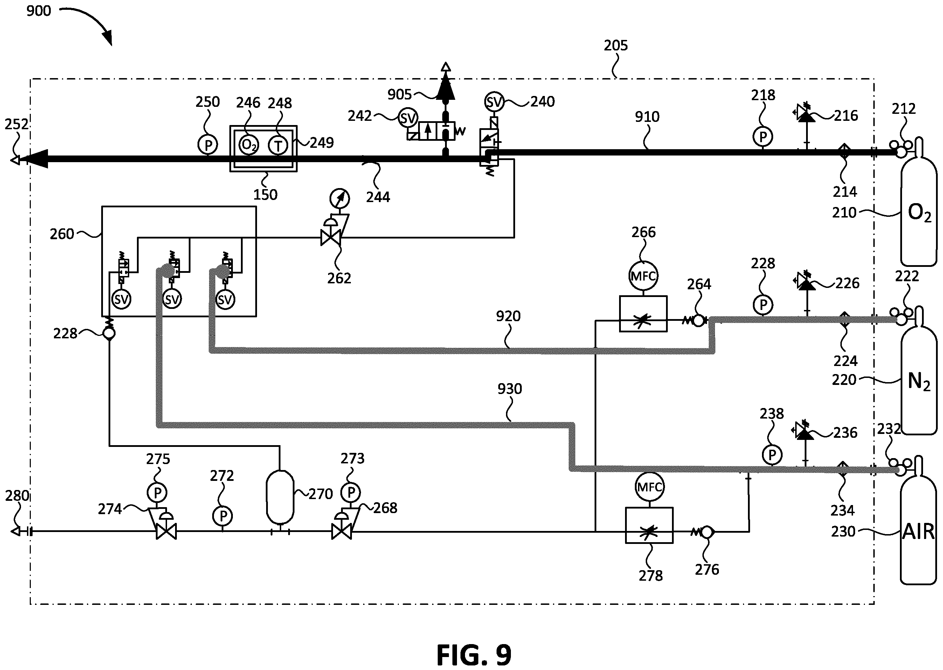

Fig. 9 illustrates an exemplary gas flow 900 for using oxygen to calibrate an oxygen sensor according to an implementation described herein. As shown in Fig. 9, before any oxygen measurements are made, gas flow 900 includes using purge flow 905 to purge any residual gases from oxygen sensor 150, oxygen gas conduit 212, gas selection valve 240, gas mixing manifold 260, and/or gas mixing manifold conduit 261 via purge valve 242. Gas flow 900 subsequently includes oxygen gas flow 910 from oxygen gas source 210 via oxygen gas conduit 212 to gas selection valve 240. Gas selection valve 240 may be controlled to select the oxygen gas from oxygen gas conduit 212 through oxygen sensor 150 to measured gas outlet 252. Nitrogen gas flow 920 may terminate at gas mixing manifold 260. Inlet air gas flow 930 may terminate at gas mixing manifold 260.

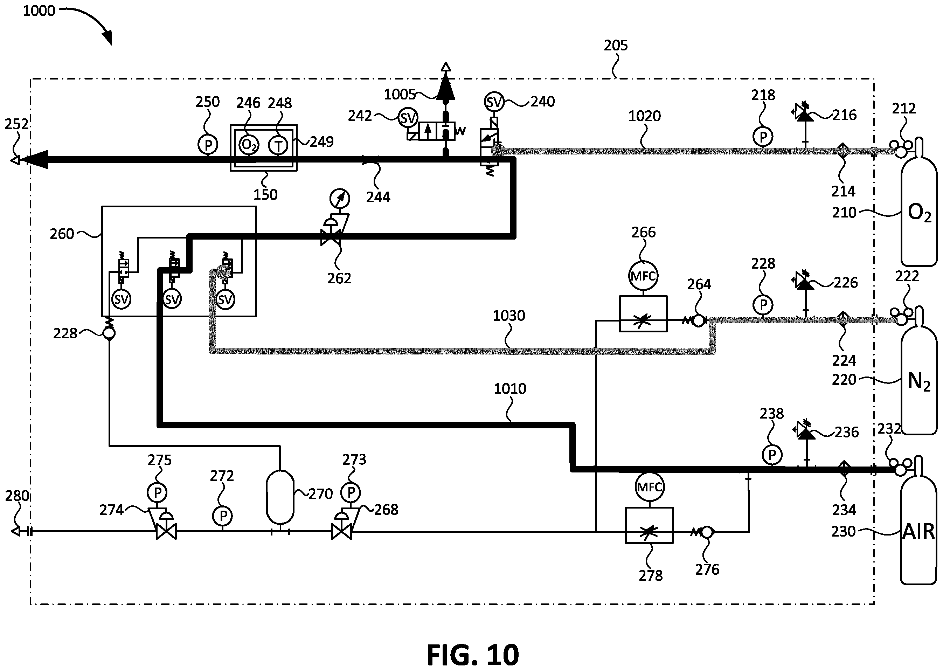

Fig. 10 illustrates an exemplary gas flow 1000 for measuring the oxygen concentration of an inlet air gas according to an implementation described herein. As shown in Fig. 10, before any oxygen measurements are made, gas flow 1000 includes using purge flow 1005 to purge any residual gases from oxygen sensor 150, oxygen gas conduit 212, gas selection valve 240, gas mixing manifold 260, and/or gas mixing manifold conduit 261 via purge valve 242. Gas flow 1000 subsequently includes inlet air gas flow 1010 from inlet air gas source 230 via second inlet air gas conduit 239 to gas mixing manifold 260. In other implementations, inlet air gas flow 1010 may intake ambient air instead of zero air gas from inlet air gas source 230. Gas mixing manifold 260 may be controlled to outlet the inlet air gas to gas mixing manifold conduit 261 and gas selection valve 240 may be controlled to select the inlet air gas from gas mixing manifold conduit 261 through oxygen sensor 150 to measured gas outlet 252. Oxygen gas flow 1020 may terminate at gas selection valve 240. Nitrogen gas flow 1030 may terminate at gas mixing manifold 260.

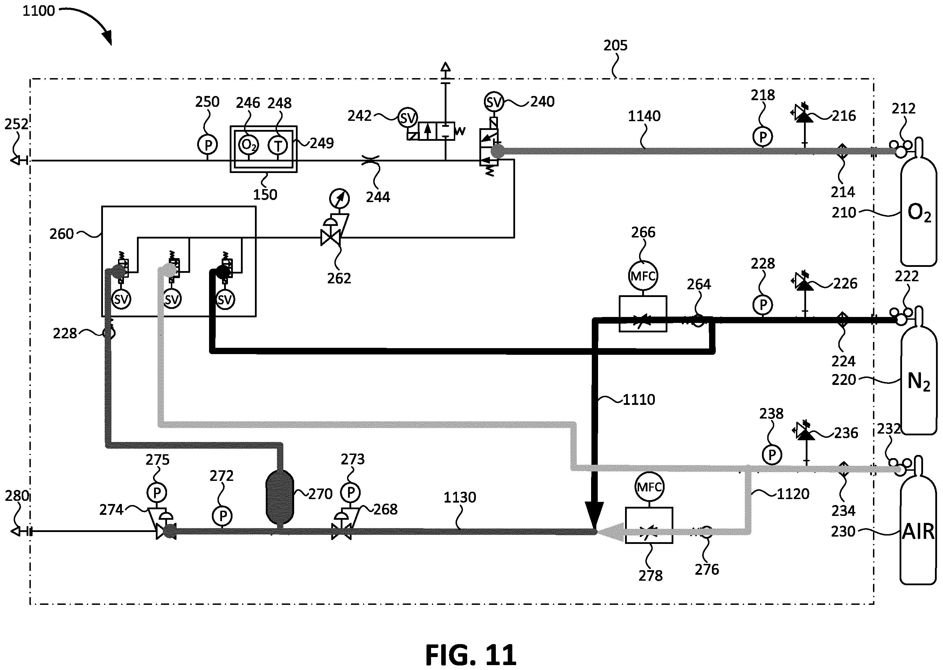

Fig. 11 illustrates an exemplary gas flow 1100 for generating a gas mixture with a particular oxygen concentration according to an implementation described herein. As shown in Fig. 11, gas flow 1100 includes nitrogen gas flow 1110 from nitrogen gas source 220 via nitrogen gas conduit 222 through mass flow controller 266 to T interconnect 267 and via second nitrogen gas conduit 229 and terminating at gas mixing manifold 260. Mass flow controller 266 may control the amount of nitrogen gas flowing to T interconnect 267 based on the target oxygen concentration and the measured oxygen concentration of inlet air gas source 230. Gas flow 1100 further includes inlet air gas flow 1120 from inlet air gas source 230 via inlet air gas conduit 232 through mass flow controller 278 to T interconnect 267 and via second inlet air gas conduit 239 and terminating at gas mixing manifold 260. In other implementations, inlet air gas flow 1120 may intake ambient air instead of zero air gas from inlet air gas source 230. Mass flow controller 278 may control the amount of inlet air gas flowing to T interconnect 267 based on the target oxygen concentration and the measured oxygen concentration of inlet air gas source 230. The nitrogen gas and the inlet air gas may mix at T interconnect 267 into a gas mixture with a target oxygen concentration to generate gas mixture gas flow 1130. Gas mixture gas flow 1130 may fill gas reservoir 270 and stop flowing at gas mixing manifold 260 and at regulator 274. Oxygen gas flow 1140 may terminate at gas selection valve 240.

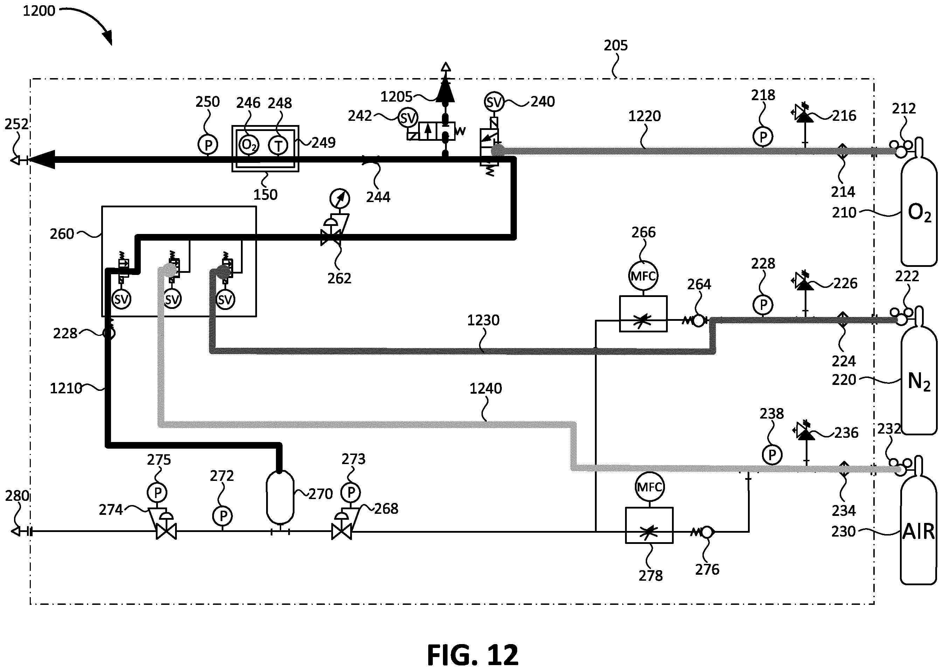

Fig. 12 illustrates an exemplary gas flow 1200 for measuring the oxygen concentration of a generated gas mixture according to an implementation described herein. As shown in Fig. 12, before any oxygen measurements are made, gas flow 1200 includes using purge flow 1205 to purge any residual gases from oxygen sensor 150, oxygen gas conduit 212, gas selection valve 240, gas mixing manifold 260, and/or gas mixing manifold conduit 261 via purge valve 242. Gas flow 1200 subsequently includes reservoir gas mixture flow 1210 from gas reservoir 270 to gas mixing manifold 260 via gas reservoir outlet conduit 271. Gas mixing manifold 260 may be controlled to outlet the reservoir gas mixture to gas mixing manifold conduit 261 and gas selection valve 240 may be controlled to select the reservoir gas mixture from gas mixing manifold conduit 261 through oxygen sensor 150 to measured gas outlet 252. Oxygen gas flow 1220 may terminate at gas selection valve 240. Nitrogen gas flow 1230 may terminate at gas mixing manifold 260. Inlet air gas flow 1240 may terminate at gas mixing manifold 260.

This application incorporates by reference the following applications: Attorney Docket No. 0080-1310PR, Application No. 63/380,004, titled "Prediction of Motor Octane Number Using a Constant Volume Combustion Chamber;" and Attorney Docket No. 0080-1311PR, Application No. 63/380,005, titled "Prediction of Research Octane Number Using a Constant Volume Combustion Chamber."

In the preceding specification, various preferred embodiments have been described with reference to the accompanying drawings. It will, however, be evident that various modifications and changes may be made thereto, and additional embodiments may be implemented, without departing from the broader scope of the invention as set forth in the claims that follow. The specification and drawings are accordingly to be regarded in an illustrative rather than restrictive sense.

For example, while series of blocks have been described with respect to Figs. 6 and 7, the order of the blocks may be modified in other implementations. Further, non-dependent blocks and/or signals may be performed in parallel.

It will be apparent that systems and/or methods, as described above, may be implemented in many different forms of software, firmware, and hardware in the implementations illustrated in the figures. The actual software code or specialized control hardware used to implement these systems and methods is not limiting of the embodiments. Thus, the operation and behavior of the systems and methods were described without reference to the specific software code--it being understood that software and control hardware can be designed to implement the systems and methods based on the description herein.

Further, certain portions, described above, may be implemented as a component that performs one or more functions. A component, as used herein, may include hardware, such as a processor, an ASIC, or a FPGA, or a combination of hardware and software (e.g., a processor executing software).

It should be emphasized that the terms "comprises" / "comprising" when used in this specification are taken to specify the presence of stated features, integers, steps, or components, but does not preclude the presence or addition of one or more other features, integers, steps, components, or groups thereof.

The term "logic," as used herein, may refer to a combination of one or more processors configured to execute instructions stored in one or more memory devices, may refer to hardwired circuitry, and/or may refer to a combination thereof. Furthermore, a logic may be included in a single device or may be distributed across multiple, and possibly remote, devices.

For the purposes of describing and defining the present invention, it is additionally noted that the term "substantially" is utilized herein to represent the inherent degree of uncertainty that may be attributed to any quantitative comparison, value, measurement, or other representation. The term "substantially" is also utilized herein to represent the degree by which a quantitative representation may vary from a stated reference without resulting in a change in the basic function of the subject matter at issue.

No element, act, or instruction used in the present application should be construed as critical or essential to the embodiments unless explicitly described as such. Also, as used herein, the article "a" is intended to include one or more items. Further, the phrase "based on" is intended to mean "based, at least in part, on" unless explicitly stated otherwise.

## Claims

1. A gas mixing system comprising:

   a first conduit to receive nitrogen gas;
   a second conduit to receive inlet air gas;
   a gas reservoir coupled to the first conduit and the second conduit;
   a gas mixing manifold coupled to the gas reservoir, the first conduit, and the second conduit;
   an oxygen sensor coupled to the gas mixing manifold; and
   a controller configured to:

      select a target oxygen concentration for a gas mixture for a combustion test;
      measure, using the oxygen sensor, an oxygen concentration in the inlet air gas received via the second conduit;
      control a nitrogen gas flow in the first conduit and an inlet air gas flow in the second conduit to fill the gas reservoir with a gas mixture having the selected target oxygen concentration, based on the measured oxygen concentration in the inlet air gas; and
      control outlet of the gas mixture from the gas reservoir for use in a combustion test.

2. The gas mixing system of claim 1, wherein the controller is further configured to:
   measure, using the oxygen sensor, an oxygen concentration of the gas mixture in the gas reservoir to determine whether the gas mixture in the gas reservoir has the selected target oxygen concentration.

3. The gas mixing system of claim 1, further comprising:

   a third conduit to receive oxygen gas; and
   a valve to select between the oxygen gas from the third conduit or a gas from the gas mixing manifold.

4. The gas mixing system of claim 3, wherein the controller is further configured to:
   calibrate the oxygen sensor using the oxygen gas from the third conduit.

5. The gas mixing system of claim 3, wherein the controller is further configured to:
   calibrate the oxygen sensor using the nitrogen gas from the first conduit.

6. The gas mixing system of claim 3, wherein the third conduit is connected to the gas reservoir, and wherein the controller is further configured to:

control the nitrogen gas flow in the first conduit and an oxygen gas flow in the third conduit to fill the gas reservoir with a synthetic air gas mixture having the selected target oxygen concentration.

7. The gas mixing system of claim 1, further comprising: a first mass flow controller to control the flow of nitrogen gas in the first conduit.

8. The gas mixing system of claim 7, further comprising:

a second mass flow controller to control the flow of inlet air gas in the second conduit; and back pressure regulator connected from the first mass flow controller and the second mass controller to the gas reservoir.

9. The gas mixing system of claim 1, further comprising: a purge valve connected to the oxygen sensor and configured to purge gas from a conduit connected to the oxygen sensor.

10. The gas mixing system of claim 1, further comprising: a pressure regulator connected to the gas reservoir and configured to control an outlet pressure for the gas reservoir.

11. A method comprising:

selecting, by a controller of a gas mixing system, a target oxygen concentration for a gas mixture for a combustion test; measuring, by the controller and using an oxygen sensor, an oxygen concentration in an inlet air gas source or ambient air source received by the gas mixing apparatus; controlling, by the controller, a nitrogen gas flow and an inlet air gas flow from the inlet air gas source or ambient air from an ambient air source to fill a gas reservoir of the gas mixing system with the gas mixture having the selected target oxygen concentration, based on the measured oxygen concentration in the inlet air gas or the ambient air; and outputting, by the controller, the gas mixture from the gas reservoir for use in the combustion test.

12. The method of claim 11, further comprising: measuring an oxygen concentration of the gas mixture in the gas reservoir to determine whether the gas mixture in the gas reservoir has the selected target oxygen concentration.

13. The method of claim 11, further comprising: calibrating the oxygen sensor using oxygen gas from an oxygen gas source.

14. The method of claim 11, further comprising: calibrating the oxygen sensor using nitrogen gas from a nitrogen gas source.

15. The method of claim 11, further comprising: controlling the nitrogen gas flow and an oxygen gas flow from an oxygen gas source to fill the gas reservoir with a synthetic air gas mixture having the selected target oxygen concentration.

**FIG. 1**

FIG. 2

**FIG. 3**

```
                400 ─────╮
                          ↓
            ┌──────────┐  ┌──────────┐  ┌──────────────────┐
            │  INPUT   │  │  OUTPUT  │  │  COMMUNICATION   │
            │  DEVICE  │  │  DEVICE  │  │    INTERFACE     │
            │   440    │  │   450    │  │       460        │
            └────┬─────┘  └────┬─────┘  └────────┬─────────┘
                 │             │                 │
    ─────────────┴─────────────┴─────────────────┴──────────── ──╮
                 │             │                           410
            ┌────┴──────┐  ┌───┴──────┐
            │ PROCESSOR │  │  MEMORY  │
            │    420    │  │   430    │
            └───────────┘  └──────────┘
```

**FIG. 4**

180

USER
INTERFACE
550

COMBUSTION
TEST MANAGER
530

ANALYZER
540

CHAMBER CONTROLLER
510

| HEAT CONTROLLER INTERFACE 512 | GAS MIXING SYSTEM INTERFACE 514 | SAMPLE INJECTOR INTERFACE 516 | COOLING SYSTEM INTERFACE 518 |

GAS MIXING SYSTEM CONTROLLER
520

| CALIBRATION MANAGER 522 | INLET AIR GAS MONITOR 524 | GAS RESERVOIR MANAGER 526 | GAS RESERVOIR MONITOR 528 |

## FIG. 5

600

610 — CALIBRATE OXYGEN SENSOR

620 — SELECT A TARGET OXYGEN CONCENTRATION FOR A GAS MIXTURE FOR A COMBUSTION TEST

630 — MEASURE OXYGEN CONCENTRATION OF INLET AIR GAS

640 — SELECT GAS MIXTURE FOR COMBUSTION TEST BASED ON SELECTED TARGET OXYGEN CONCENTRATION AND MEASURED OXYGEN CONCENTRATION OF INLET AIR GAS

650 — FILL GAS RESERVOIR WITH SELECTED GAS MIXTURE

660 — VERIFY GAS MIXTURE IN GAS RESERVOIR

670 — USE RESERVOIR GAS MIXTURE TO PERFORM COMBUSTION TEST

**FIG. 6**

700

710 — MEASURE OXYGEN CONCENTRATION IN GAS RESERVOIR

720 — MEASURE PRESSURE IN GAS RESERVOIR

730 — CALCULATE OXYGEN CONTENT IN GAS RESERVOIR BASED ON MEASURED OXYGEN CONCENTRATION AND PRESSURE

740 — SELECT TARGET OXYGEN CONCENTRATION IN GAS RESERVOIR

750 — SELECT TARGET PRESSURE IN GAS RESERVOIR

760 — DETERMINE REQUIRED AMOUNTS OF OXYGEN AND NITROGEN TO ACHIEVE TARGET OXYGEN CONCENTRATION AT TARGET PRESSURE IN GAS RESERVOIR

770 — DETERMINE MIXING RATIO OF INLET AIR AND NITROGEN BASED ON THE DETERMINED REQUIRED AMOUNTS OF OXYGEN AND NITROGEN

780 — FILL GAS RESERVOIR USING THE DETERMINED MIXING RATIO OF INLET AIR AND NITROGEN

790 — VERIFY GAS MIXTURE IN GAS RESERVOIR

795 — USE RESERVOIR GAS MIXTURE TO PERFORM COMBUSTION TEST

**FIG. 7**

**FIG. 8**

FIG. 9

EP 4 506 057 A1

**FIG. 10**

EP 4 506 057 A1

**FIG. 11**

FIG. 12

Europäisches
Patentamt
European
Patent Office
Office européen
des brevets

# EUROPEAN SEARCH REPORT

**Application Number**

EP 24 19 3241

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 102 608 257 A (UNIV WUHAN TECH) 25 July 2012 (2012-07-25) | 1-3,6, 11,12,15 | INV. B01F23/10 |
| Y | * paragraphs [0026] - [0031]; figure * | 4,5, 7-10,13, 14 | G01N33/28 |
| Y | DE 10 2004 022988 A1 (ENGL BERNHARD [DE]; BOHN HANS JOERG [DE]) 1 December 2005 (2005-12-01) * paragraphs [0024] - [0042]; figures 1, 2 * | 4,5,9, 13,14 | |
| Y | US 2005/247311 A1 (VACCHIANO CHARLES [US] ET AL) 10 November 2005 (2005-11-10) * paragraph [0170]; figure 1 * | 7,8,10 | |
| A | CN 208 771 224 U (WUHAN RUIHUA INSTR & EQUIPMENT CO LTD) 23 April 2019 (2019-04-23) * claim 1; figure * | 1-15 | |

TECHNICAL FIELDS SEARCHED (IPC)

B01F
G01N
A61M

The present search report has been drawn up for all claims

| Place of search | Date of completion of the search | Examiner |
|---|---|---|
| The Hague | 29 November 2024 | Wilhelm-Shalganov, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 19 3241

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2024

| Patent document cited in search report | | Publication date | Patent family member(s) | Publication date |
|---|---|---|---|---|
| CN 102608257 | A | 25-07-2012 | NONE | |
| DE 102004022988 | A1 | 01-12-2005 | NONE | |
| US 2005247311 | A1 | 10-11-2005 | NONE | |
| CN 208771224 | U | 23-04-2019 | NONE | |

EPO FORM P0459

For more details about this annex : see Official Journal of the European Patent Office, No. 12/82

**REFERENCES CITED IN THE DESCRIPTION**

**Patent documents cited in the description**

- US 63380004 B
- WO 63380005 A